# FASCICULE DE BREVET EUROPEEN

(11) **EP 2 400 946 B1**
(45) Date de publication et mention de la délivrance du brevet: **24.10.2018**
(21) Numéro de dépôt: 10710089.3
(22) Date de dépôt: 26.02.2010
(51) Int. Cl.: A61K 8/34, A61Q 5/06, A61Q 5/10, A61K 8/96, A61K 8/49

(54) **COMPOSITION COMPRENANT UN COLORANT ET LE 3-PHENYL-1-PROPANOL, COLORATION DE FIBRES KERATINIQUES**
ZUSAMMENSETZUNG MIT EINEM FARBSTOFF UND 3-PHENYL-1-PROPANOL SOWIE FÄRBUNG VON KERATINFASERN
COMPOSITION CONTAINING A DYE AND 3-PHENYL-1-PROPANOL AND DYEING OF KERATIN FIBRES

(30) Priorité: 27.02.2009 FR 0951283; 27.02.2009 FR 0951285; 27.02.2009 FR 0951287
(43) Date de publication de la demande: 04.01.2012
(73) Titulaire: L'Oréal, 75008 Paris (FR)
(72) Inventeur: LAGRANGE, Alain, F-77700 Coupvray (FR); LALLEMAN, Boris, F-75004 Paris (FR)
(74) Mandataire: Kuhlmann, Sonia
(86) Numéro de dépôt international: PCT/FR2010/050338
(87) Numéro de publication internationale: WO 2010/097559

(56) Documents cités:
- EP-A1- 1 849 454
- EP-A2- 1 175 893
- EP-A2- 1 714 637
- FR-A1- 2 776 289
- FR-A1- 2 893 027
- JP-A- 10 245 327
- JP-A- 2007 008 892
- US-A- 3 630 654
- "International Cosmetic Ingredient Dictionary and Handbook", 2006, The Cosmetic, Toiletry, and Fragrance Association, Washington DC, XP002561510, vol. 2, pages 1743-1743, page 1743

## Description

La présente invention a pour objet des compositions de coloration de fibres kératiniques humaines, comprenant dans un milieu cosmétiquement acceptable, au moins un colorant choisi parmi les colorants directs synthétiques non ioniques ou cationiques et les colorants naturels et le 3-phényl-1-propanol.

On connaît deux grands modes de coloration des fibres kératiniques humaines, et en particulier les cheveux.

Le premier, appelé coloration d'oxydation ou permanente, consiste à mettre en oeuvre un ou plusieurs précurseurs de colorant d'oxydation, plus particulièrement une ou plusieurs bases d'oxydation éventuellement associées à un ou plusieurs coupleurs.

Habituellement, des bases d'oxydation sont choisies parmi les ortho- ou paraphénylènediamines, les ortho- ou para-aminophénols ainsi que des composés hétérocycliques. Ces bases d'oxydation sont des composés incolores ou faiblement colorés qui, associés à des produits oxydants, permettent d'accéder, par un processus de condensation oxydative, à des espèces colorées qui restent piégées à l'intérieur de la fibre.

Bien souvent, on fait varier les nuances obtenues avec ces bases d'oxydation en les associant à un ou plusieurs coupleurs, ces derniers étant choisis notamment parmi les métadiamines aromatiques, les méta-aminophénols, les méta-diphénols et certains composés hétérocycliques, tels que des composés indoliques.

La variété des molécules mises en jeu au niveau des bases d'oxydation et des coupleurs permet l'obtention d'une riche palette de couleurs.

La plupart des colorants d'oxydation mis en oeuvre ont une solubilité en milieu aqueux suffisante et il existe maintenant de nombreux supports de coloration adaptés pour les recevoir. Cependant les supports de coloration d'oxydation existant ne donnent pas totalement satisfaction notamment vis-à-vis de la puissance ou de l'intensité des colorations obtenues ou de leur chromaticité ou de leur sélectivité (différence de coloration obtenue entre les zones plus ou moins sensibilisées d'une chevelure).

Le deuxième mode de coloration, appelé coloration directe ou semi-permanente, comprend l'application de colorants directs qui sont des molécules colorées et colorantes, ayant une affinité pour les fibres, d'origine naturelle ou synthétique. Etant donnée la nature des molécules employées, celles-ci restent plutôt en surface de la fibre et pénètrent relativement peu à l'intérieur de la fibre, comparées aux petites molécules de précurseurs de colorants d'oxydation.

Les colorants directs généralement employés sont choisis parmi les colorants directs nitrés benzéniques, anthraquinoniques, nitropyridiniques, azoïques, méthiniques, azométhiniques, xanthéniques, acridiniques, aziniques ou triarylméthaniques. Les espèces chimiques mises en oeuvre peuvent être non ioniques, anioniques (colorants acides) ou cationiques (colorants basiques).

Les colorants directs synthétiques nécessitent souvent pour leur mise en oeuvre dans des compositions tinctoriales et leur application sur les fibres kératiniques l'utilisation de solvants. L'alcool benzylique est très largement utilisé.

Cependant les compositions comprenant des colorants directs synthétiques non ioniques ou cationiques avec un solvant comme l'alcool benzylique ne donnent pas totalement satisfaction notamment vis-à-vis de la puissance ou de l'intensité des colorations obtenues ou de leur chromaticité ou de leur sélectivité (différence de coloration obtenue entre les zones plus ou moins sensibilisés d'une chevelure).

La situation est différente dans le cas de colorants naturels.

En effet, pour colorer efficacement les fibres kératiniques, certains de ces colorants doivent être dans la majeure partie des cas, employés en présence de solvants dont le rôle est de les vectoriser dans la fibre. Parmi les solvants cosmétiques connus pour cet effet, on utilise fréquemment les solvants aromatiques. On peut citer plus particulièrement l'alcool benzylique, le benzyloxyéthanol ou encore le phénoxyéthanol.

Néanmoins, la présence d'alcools aromatiques dans ces formulations amplifie le problème de tachage du cuir chevelu et de la peau rencontré en coloration.

En outre, et cela représente un autre inconvénient à l'utilisation de tels colorants, la présence des solvants aromatiques, très peu solubles en milieu aqueux, nécessite l'emploi de quantités importantes de co-solvants (habituellement l'éthanol) pour les rendre compatibles avec les formulations tinctoriales classiques.

L'un des objectifs de la présente invention est donc de proposer des compositions tinctoriales comprenant au moins un colorant direct synthétique non ionique ou cationique, ou au moins un colorant naturel, ou leurs combinaisons, et qui présentent des propriétés tinctoriales satisfaisantes, notamment en ce qu'elles permettent d'obtenir des colorations puissantes, homogènes entre la pointe et la racine d'une même fibre et d'une fibre à l'autre, et chromatiques, même sur des cheveux sensibilisés.

Ces objectifs et d'autres sont atteints par la présente invention qui a pour objet des compositions tinctoriales comprenant dans un milieu cosmétiquement acceptable, un ou plusieurs colorants choisis parmi les colorants directs synthétiques non ioniques ou cationiques, les colorants naturels, ou leurs mélanges, et le 3-phényl-1-propanol.

D'autres caractéristiques et avantages de l'invention apparaîtront plus clairement à la lecture de la description et des exemples.

Il est à noter que dans ce qui va suivre, et à moins d'une autre indication, les bornes d'un domaine de valeurs sont comprises dans ce domaine.

La composition comprend un ou plusieurs colorants directs synthétiques choisis parmi des espèces cationiques ou non ioniques.

Par colorants synthétiques, on entend tout colorant dont la structure chimique ne se trouve pas dans la nature.

A titre d'exemples de colorants directs synthétiques convenables, on peut citer les colorants directs azoïques ; méthiniques ; carbonyles ; aziniques ; nitrés (hétéro)aryle ; tri-(hétéro)aryle méthanes ; les porphyrines et les phtalocyanines, seuls ou en mélanges. A titre d'exemples de colorants directs synthétiques convenables, on peut citer les colorants directs azoïques ; méthiniques ; carbonyles ; aziniques ; nitrés (hétéro)aryle ; tri-(hétéro)aryle méthanes ; seuls ou en mélanges.

Plus particulièrement, les colorants azoïques comprennent une fonction -N=N- dont les deux atomes d'azote ne sont pas simultanément engagés dans un cycle. Il n'est toutefois pas exclu que l'un des deux atomes d'azote de l'enchaînement -N=N- soit engagé dans un cycle.

Les colorants de la famille des méthines sont plus particulièrement des composés comprenant au moins un enchaînement choisi parmi >C=C< et -N=C< dont les deux atomes ne sont pas simultanément engagés dans un cycle. Il est toutefois précisé que l'un des atomes d'azote ou de carbone des enchaînements peut être engagé dans un cycle. Plus particulièrement, les colorants de cette famille sont issus de composés de type méthine vraie (comprenant un ou plusieurs enchaînements -C=C- précités) ; azométhine (comprenant au moins un ou plusieurs enchaînements -C=N-) avec par exemple les azacabocyanines et leurs isomères, les diazacarbocyanines et leurs isomères, les tétraazacarbocyanines ; mono- et di- arylméthane ; indoamines (ou diphénylamines) ; indophénols ; indoanilines.

Concernant les colorants de la famille des carbonyles, on peut citer par exemple les colorants choisis parmi les acridone, benzoquinone, anthraquinone, naphtoquinone, benzanthrone, anthranthrone, pyranthrone, pyrazolanthrone, pyrimidinoanthrone, flavanthrone, idanthrone, flavone, (iso)violanthrone, isoindolinone, benzimidazolone, isoquinolinone, anthrapyridone, pyrazoloquinazolone, périnone, quinacridone, quinophthalone, indigoïde, thioindigo, naphtalimide, anthrapyrimidine, dicétopyrrolopyrrole, coumarine.

Concernant les colorants de la famille des aziniques, on peut citer notamment les azine, xanthène, thioxanthène, fluorindine, acridine, (di)oxazine, (di)thiazine, pyronine.

Les colorants nitrés (hétéro)aromatiques sont plus particulièrement des colorants directs nitrés benzéniques ou nitrés pyridiniques.

Concernant les colorants de type porphyrines ou phtalocyanines, on peut mettre en oeuvre des composés cationiques ou non, comprenant éventuellement un ou plusieurs métaux ou ions métalliques, comme par exemple des métaux alcalins et alcalino-terreux, le zinc et le silicium.

A titre d'exemple de colorants directs de synthèse particulièrement convenables, on peut citer les colorants nitrés de la série benzénique ; les colorants directs azoïques ; méthiniques ; azométhiniques avec plus particulièrement les diazacarbocyanines et leurs isomères et les tétraazacarbocyanines (tétraazapentaméthines) ; les colorants directs quinoniques et en particulier anthraquinoniques, naphtoquinoniques ou benzoquinoniques ; les colorants directs aziniques ; xanthéniques ; triarylméthaniques ; indoaminiques ; indigoïdes ; phtalocyanines et porphyrines ; seuls ou en mélanges.

De préférence, les colorants directs sont choisis parmi les colorants nitrés de la série benzénique ; azoïques ; azométhines avec les diazacarbocyanines et leurs isomères, les tétraazacarbocyanines (tétraazapentaméthines) ; les colorants directs anthraquinoniques ; les colorants directs triarylméthaniques ; seuls ou en mélanges.

De manière encore plus préférée, ces colorants directs additionnels sont choisis parmi les colorants nitrés de la série benzénique ; les colorants directs azoïques ; azométhines avec les diazacarbocyanines et leurs isomères, les tétraazacarbocyanines (tétraazapentaméthines) ; seuls ou en mélange.

Ces colorants peuvent être des colorants monochromophoriques (c'est-à-dire ne comprenant qu'un seul colorant) ou polychromophoriques, de préférence di- ou trichromophoriques ; les chromophores pouvant être identiques ou non, de la même famille chimique ou non. A noter que qu'un colorant polychromophorique comprend plusieurs radicaux issus chacun d'une molécule absorbant dans le domaine visible entre 400 et 800 nm. De plus cette absorbance du colorant ne nécessite ni oxydation préalable de celui-ci, ni association avec d'autre(s) espèce(s) chimique(s).

Dans le cas de colorants polychromophoriques, les chromophores sont reliés entre eux au moyen d'au moins un bras de liaison qui peut être cationique ou non.

Parmi les colorants polychromophoriques, on peut citer plus particulièrement les colorants di- ou tri- chromophoriques, de préférence dichromophoriques, azoïques et/ou azométhiniques, symétriques ou non, comprenant d'une part au moins un hétérocycle aromatique comprenant 5 ou 6 chaînons, éventuellement condensé, comprenant au moins un atome d'azote quaternisé engagé dans ledit hétérocycle et éventuellement au moins un autre hétéroatome (tel que l'azote, le soufre, l'oxygène), et d'autre part, au moins un groupement phényle ou naphtyle, éventuellement substitué, éventuellement porteur d'au moins un groupement OR avec R représentant un atome d'hydrogène, un radical alkyle éventuellement substitué en C₁-C₆, un noyau phényle éventuellement substitué, ou d'au moins un groupement N(R')₂ avec R' identiques ou non, représentant un atome d'hydrogène, un radical alkyle éventuellement substitué en C₁-C₆, un noyau phényle éventuellement substitué ; les radicaux R' pouvant former avec l'atome d'azote auquel ils sont liés, un hétérocycle saturé à 5 ou 6 chaînons, ou bien encore l'un et/ou les deux radicaux R' peuvent former chacun avec l'atome de carbone du cycle aromatique placé en ortho de l'atome d'azote, un hétérocycle saturé à 5 ou 6 chaînons.

A titre d'hétérocycle cationique aromatique, on peut citer de préférence, les cycles à 5 ou 6 chaînons comprenant 1 à 3 atomes d'azote, de préférence 1 ou 2 atomes d'azote, l'un étant quaternisé ; ledit hétérocycle étant par ailleurs éventuellement condensé à un noyau benzénique. Il est à noter de même que l'hétérocycle peut éventuellement comprendre un autre hétéroatome différent de l'azote, comme le soufre ou l'oxygène.

Si les hétérocycles ou groupements phényle ou napthyle sont substitués, ils le sont par exemple par un ou plusieurs radicaux alkyle en C₁-C₈ éventuellement substitués par un groupement hydroxy, alcoxy en C₁-C₂, hydroxyalcoxy en C₂-C₄, acétylamino, amino substitué par un ou deux radicaux alkyle en C₁-C₄, éventuellement porteurs d'un groupement hydroxyle ou les deux radicaux pouvant former avec l'atome d'azote auquel ils sont rattachés, un hétérocycle à 5 ou 6 chaînons, comprenant éventuellement un autre hétéroatome identique ou différent de l'azote ; un atome d'halogène ; un groupement hydroxyle; un radical alcoxy en C₁-C₂ ; un radical hydroxyalcoxy en C₂-C₄; un radical amino ; un radical amino substitué par un ou deux radicaux alkyle, identiques ou différents, en C₁-C₄ éventuellement porteurs d'un groupement hydroxyle.

De préférence, le bras de liaison est une chaîne alkyle en C₁-C₂₀, linéaire, ramifiée ou cyclique ; éventuellement interrompue par au moins un hétéroatome (tel que l'azote, l'oxygène) et/ou par au moins un groupe en comprenant (CO, SO₂); éventuellement interrompue par au moins un groupement phényle ou naphtyle substitué ou non ; éventuellement interrompue par au moins un hétérocycle saturé, insaturé ou aromatique, condensé ou non avec un noyau phényle, ledit hétérocycle comprenant au moins un atome d'azote quaternisé engagé dans ledit cycle et éventuellement au moins un autre hétéroatome (tel que l'oxygène, l'azote ou le soufre) ; éventuellement interrompue par au moins un groupement ammonium quaternaire substitué par deux groupements alkyle en C₁-C₁₅ ; le bras de liaison ne comprenant pas de groupement nitro, nitroso ou peroxo.

Si les hétérocycles ou noyaux aromatiques sont substitués, ils le sont par exemple par un ou plusieurs radicaux alkyle en C₁-C₈ éventuellement substitués par un groupement hydroxy, alcoxy en C₁-C₂, hydroxyalcoxy en C₂-C₄, acétylamino, amino substitué par un ou deux radicaux alkyle, identiques ou différents, en C₁-C₄, éventuellement porteurs d'un groupement hydroxyle ou les deux radicaux pouvant former avec l'atome d'azote auquel ils sont rattachés, un hétérocycle à 5 ou 6 chaînons, comprenant éventuellement un autre hétéroatome identique ou différent de l'azote ; un atome d'halogène ; un groupement hydroxyle; un radical alcoxy en C₁-C₂; un radical hydroxyalcoxy en C₂-C₄; un radical amino ; un radical amino substitué par un ou deux radicaux alkyle, identiques ou différents, en C₁-C₄ éventuellement porteurs d'un groupement hydroxyle.

La liaison entre le bras de liaison et chaque chromophore se fait en général au moyen d'un hétéroatome substituant le noyau phényle ou napthyle ou au moyen de l'atome d'azote quaternisé de l'hétérocycle cationique.

Parmi les colorants directs monochromophoriques azoïques, azométhines, méthines utilisables selon l'invention on peut citer les colorants cationiques décrits dans les demandes de brevets WO 95/15144, WO 95/01772 et EP 714954; FR 2189006, FR 2285851, FR 2140205, EP 1378544, EP 1674073.

Ainsi, on peut tout notamment citer les colorants directs cationiques correspondants aux formules suivantes : 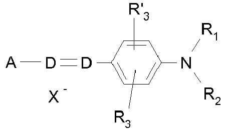 dans laquelle :
D représente un atome d'azote ou le groupement -CH,
R₁ et R₂, identiques ou différents, représentent un atome d'hydrogène ; un radical alkyle en C₁-C₄ pouvant être substitué par un radical -CN, -OH ou -NH₂ ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
R₃ et R'₃, identiques ou différents, représentent un atome d'hydrogène ou d'halogène choisi parmi le chlore, le brome, l'iode et le fluor, un radical cyano, alkyl en C₁-C₄, alcoxy en C₁-C₄ ou acétyloxy,
X⁻ représente un anion de préférence choisi parmi le chlorure, le méthyl sulfate et l'acétate, A représente un groupement choisi par les structures suivantes : 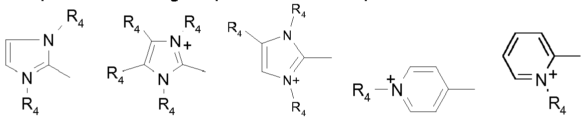
dans lesquelles R₄ représente un radical alkyle en C₁-C₄ pouvant être substitué par un radical hydroxyle; 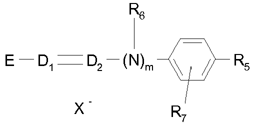
dans lesquelles :
   R₅ représente un atome d'hydrogène, un radical alcoxy en C₁-C₄, un atome d'halogène tel que le brome, le chlore, l'iode ou le fluor,
   R₆ représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
   R₇ représente un atome d'hydrogène ou d'halogène tel que le brome, le chlore, l'iode ou le fluor,
   D₁ et D₂, identiques ou différents, représentent un atome d'azote ou le groupement -CH,
      m = 0 ou 1,
   X⁻ représente un anion cosmétiquement acceptable et de préférence choisi parmi le chlorure, le méthyl sulfate et l'acétate,
   E représente un groupement choisi par les structures suivantes : 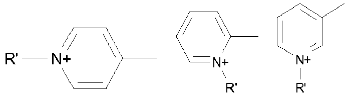
   dans lesquelles R' représente un radical alkyle en C₁-C₄ ;
   lorsque m = 0 et que D₁ représente un atome d'azote, alors E peut également désigner un groupement de structure suivante : 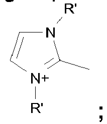
   dans laquelle R' représente un radical alkyle en C₁-C₄.

Parmi les composés précités, on utilise tout particulièrement les composés suivants :

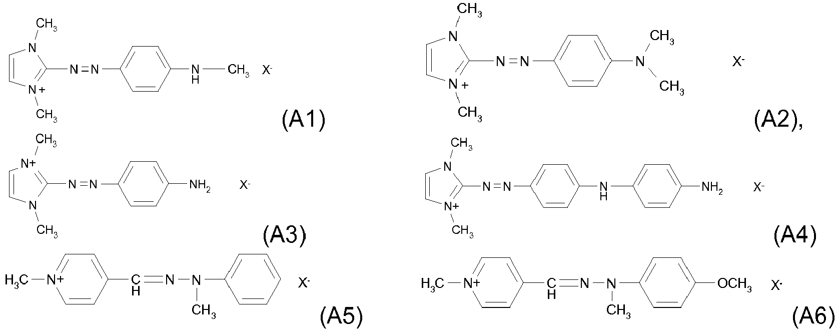

Parmi les colorants de type tétraazapentaméthiniques utilisables selon l'invention, on peut citer les composés suivants figurant dans le tableau ci-dessous

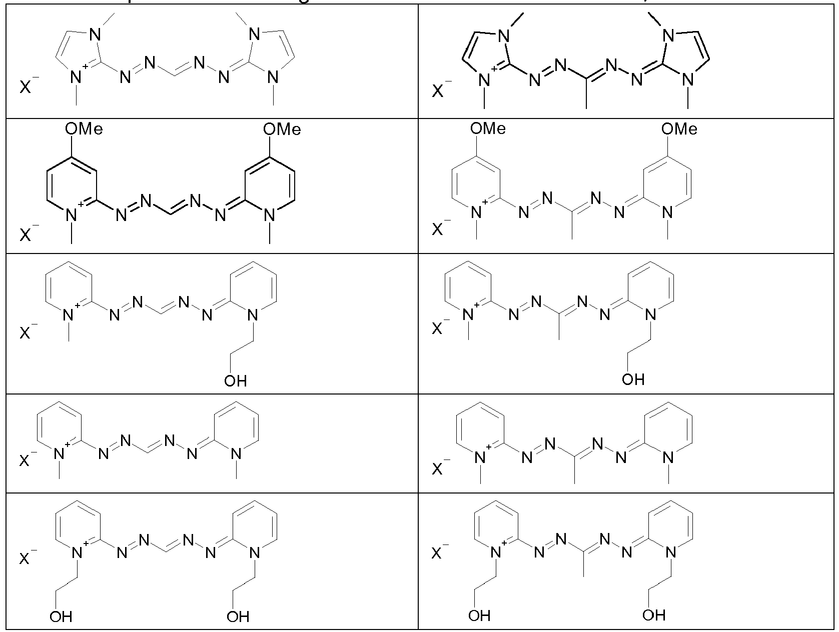

X⁻ représente un anion de préférence choisi parmi le chlorure, l'iodure, le méthyl sulfate, l'éthyl sulfate, l'acétate.

Parmi les colorants directs nitrés benzéniques utilisables selon l'invention, on peut citer de manière non limitative les composés suivants :
- 1,4-diamino-2-nitrobenzène
- 1-amino-2 nitro-4-β-hydroxyéthylaminobenzène
- 1-amino-2 nitro-4-bis(β-hydroxyéthyl)-aminobenzène
- 1,4-Bis(β-hydroxyéthylamino)-2-nitrobenzène
- 1-β-hydroxyéthylamino-2-nitro-4-bis-(β-hydroxyéthylamino)-benzène
- 1-β-hydroxyéthylamino-2-nitro-4-aminobenzène
- 1-β-hydroxyéthylamino-2-nitro-4-(éthyl)(β-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-Diamino-4-nitrobenzène
- 1-amino-2-β-hydroxyéthylamino-5-nitrobenzène
- 1,2-Bis-(β-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1- β-hydroxyéthyloxy-2-β-hydroxyéthylamino-5-nitrobenzène
- 1-Méthoxy-2-β-hydroxyéthylamino-5-nitrobenzène
- 1-β-hydroxyéthyloxy-3-méthylamino-4-nitrobenzène
- 1-β□γ-dihydroxypropyloxy-3-méthylamino-4-nitrobenzène
- 1-β-hydroxyéthylamino-4-β,γ-dihydroxypropyloxy-2-nitrobenzène
- 1-β□γ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-3-hydroxyéthylamino-3-nitrobenzène.

Comme autres colorants utilisables selon l'invention on peut également citer parmi les colorants directs azoïques les colorants suivants, décrits dans le COLOUR INDEX INTERNATIONAL 3e édition :
- Disperse Red 17
- Disperse Red 13
- Basic Red 22
- Basic Red 76
- Basic Yellow 57
- Basic Brown 16
- Basic Brown 17
- Disperse Green 9
- Disperse Black 9.
- Solvent Black 3
- Disperse Blue 148
- Disperse Violet 63
- Solvent Orange 7

On peut aussi citer le 1-(4'-aminodiphénylazo)-2-méthyl-4bis-(β-hydroxyéthyl) aminobenzène (Nom INCI : HC Yellow 7).

Parmi les colorants directs quinoniques on peut citer les colorants suivants :
- Disperse Red 15
- Solvent Violet 13
- Solvent Blue 14
- Disperse Violet 1
- Disperse Violet 4
- Disperse Blue 1
- Disperse Violet 8
- Disperse Blue 3
- Disperse Red 11
- Disperse Blue 7
- Disperse Blue 14
- Basic Blue 22
- Disperse Violet 15
- Disperse Blue 377
- Disperse Blue 60
- Basic Blue 99
ainsi que les composés suivants :
- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(βγ-dihydroxypropylamino)-anthraquinone.

On peut aussi citer la coumarine Disperse Yellow 82. Parmi les colorants aziniques on peut citer les composés suivants :
- Basic Blue 17
- Basic Red 2
- Solvent Orange 15.

Parmi les colorants triarylméthaniques utilisables selon l'invention, on peut citer les composés suivants :
- Basic Green 1
- Basic Violet 3
- Basic Violet 14
- Basic Blue 7
- Basic Blue 26

Parmi les colorants indoaminiques utilisables selon l'invention, on peut citer les composés suivants :
- 2-β-hydroxyéthylamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine.

De préférence, les colorants directs sont choisis parmi les colorants directs monochromophoriques de types nitrés benzéniques ; azoïques ; azométhines avec les diazacarbocyanines et leurs isomères, les tétraazacarbocyanines (tétraazapentaméthines) ; les colorants directs anthraquinoniques ; les colorants directs triarylméthaniques ; et encore plus particulièrement parmi les colorants nitrés benzéniques ; azoïques ; azométhines avec les diazacarbocyanines et leurs isomères, les tétraazacarbocyanines (tétraazapentaméthines) ; ces colorants étant présents seuls ou en mélange. De préférence, les colorants directs cationiques sont choisis parmi les colorants directs monochromophoriques de types azoïques ; méthines ; azométhines avec les diazacarbocyanines et leurs isomères, les tétraazacarbocyanines (tétraazapentaméthines) ; anthraquinoniques ; seuls ou en mélange.

Pour les colorants non ioniques on préfère tout particulièrement les composés présentant un LogP supérieur ou égal à 2.

En ce qui concerne les colorants directs synthétiques de LogP supérieur ou égal à 2, il est rappelé que la valeur du logP représente de façon classique le coefficient de partage du colorant entre l'octanol et l'eau. Le logP peut être calculé selon la méthode décrite dans l'article de Meylan et Howard « Atom / Fragment contribution method for estimating octanol-water partition coefficient », J. Pharm. Sci. 84 :83-92, 1995. Cette valeur peut aussi être calculée à partir de nombreux logiciels disponibles sur le marché qui détermine le logP en fonction de la structure d'une molécule. A titre d'exemple, on peut citer le logiciel Epiwin de l'agence de l'environnement des Etats-Unis.

En particulier, les colorants convenant à la mise en oeuvre de l'invention sont choisis parmi les composés suivants, seuls ou en mélange :

| **Colorant** | **Structure chimique** | **logP** |
|---|---|---|
| Disperse Red 17 | 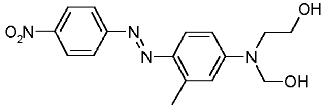 | 3.69 |
| Disperse Violet 1 | 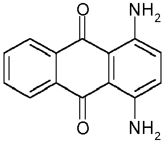 | 3.0 |
| HC Yellow 7 | 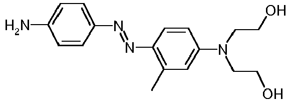 | 2.38 |
| Disperse Blue 377 | 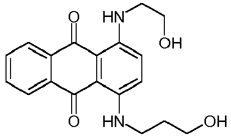 | 3.21 |
| Disperse Red 13 | 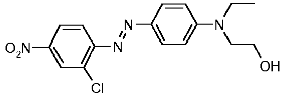 | 5.22 |
| Disperse Green 9 | 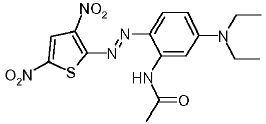 | 4.23 |
| Solvent Black 3 | 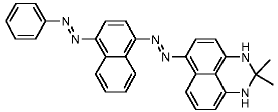 | 7.50 |
| Disperse Blue 148 | 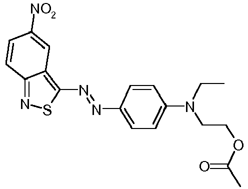 | 4.81 |
| Disperse Violet 63 | 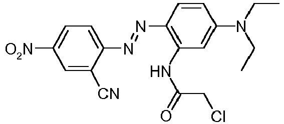 | 5.30 |
| Disperse Blue 60 | 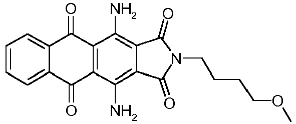 | 3.38 |
| Disperse Blue 14 | 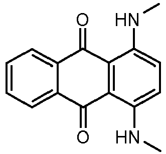 | 4.25 |
| Solvent Orange 15 | 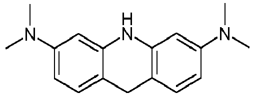 | 3.90 |
| Solvent Orange 7 | 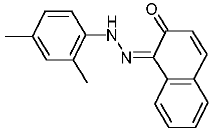 | 4.40 |
| Solvent Blue 14 | 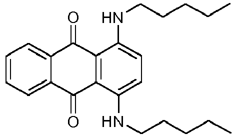 | 8.18 |
| Disperse Yellow 82 | 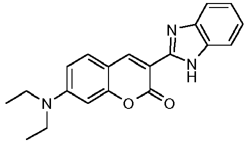 | 3.68 |

Encore plus préférentiellement, les colorants directs synthétiques de l'invention sont choisis parmi les colorants (A1) à (A6) cités précédemment et les colorants non ioniques de logP supérieur ou égal à 2.

Lorsqu'ils sont présents, le ou les colorants directs de synthèse représentent de 0,0001 à 10 % en poids par rapport au poids de la composition, et de préférence de 0,005 à 5 % en poids par rapport à la même référence.

Comme indiqué précédemment, la composition tinctoriale selon l'invention peut comprendre un ou plusieurs colorants naturels.

Par colorants naturels, on entend tout colorant ou précurseur de colorant ayant une occurrence naturelle et produit soit par extraction (et éventuellement purification) depuis une matrice végétale, soit par synthèse chimique.

Le ou les colorants naturels convenables en particulier à la mise en oeuvre de l'invention sont choisis de préférence parmi la lawsone, la juglone, l'alizarine, la purpurine, l'acide carminique, l'acide kermésique, la purpurogalline, l'anthragallol, la protocatéchaldéhyde, l'indigo, l'isatine, la curcumine, la spinusoline, les chlorophylles, les chlorophyllines, les orcéines, l'hématéine, l'hématoxyline, la braziline, la braziléine, les colorants du carthame (comme par exemple la carthamine), les flavonoïdes (avec par exemple la morine, l'apigénidine, le santal), les anthocyanes (du type de l'apigéninidine), les caroténoïdes, les tanins, le sorgho et le carmin de cochenille, ou leurs mélanges.

On peut également utiliser les extraits ou décoctions contenant ces colorants naturels et notamment les extraits à base de henné.

De préférence, le ou les colorants naturels sont choisis parmi la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine, la chlorophylline, le sorgho, les orcéines, et le carmin de cochenille, ou leurs mélanges.

Le ou les colorant naturels représentent plus particulièrement de 0,001 à 10% en poids, de préférence de 0,01 à 8 % en poids, encore plus préférentiellement de 0,1 à 5% en poids par rapport au poids de la composition.

La composition selon l'invention peut également comprendre un ou plusieurs colorants additionnels synthétiques anioniques, en particulier tels que ceux décrits dans le COLOUR INDEX INTERNATIONAL 3e édition sous l'appellation ACID et en particulier :
- Disperse Red 17
- Acid Yellow 9
- Acid Black 1
- Acid Yellow 36
- Acid Orange 7
- Acid Red 33
- Acid Red 35
- Acid Yellow 23
- Acid Orange 24
- Acid Violet 43
- Acid Blue 62
- Acid blue 9
- Acid Violet 49
- Acid Blue 7.

Lorsqu'ils sont présents, le ou les colorants directs additionnels synthétiques anioniques représentent de 0,0001 à 10 % en poids par rapport au poids de la composition, et de préférence de 0,005 à 5 % en poids par rapport à la même référence.

La composition tinctoriale selon l'invention peut également comprendre un ou plusieurs agents de conditionnement.

A titre d'exemple, on peut citer les silicones linéaires, cycliques, ramifiées ou non ramifiées, volatiles ou non volatiles. Ces silicones peuvent se présenter sous forme d'huiles, de résines ou de gommes, elles peuvent en particulier être des polyorganosiloxanes insolubles dans le milieu cosmétiquement acceptable.

Les organopolysiloxanes sont définis plus en détail dans l'ouvrage de Walter NOLL "Chemistry and Technology of Silicones" (1968) Academic Press. Ils peuvent être volatils ou non volatils.

Lorsqu'elles sont volatiles, les silicones sont plus particulièrement choisies parmi celles possédant un point d'ébullition compris entre 60° C et 260° C.,

A titre d'agent de conditionnement, on peut aussi utiliser les polymères cationiques tels que les polyquaterniums 22, 6, 10, 11, 35 et 37 et le chlorure d'hexadimethrine.

La concentration en agent(s) de conditionnement dans la ou les compositions utiles dans l'invention peut varier de 0,01 à 10% en poids par rapport au poids total de la composition, de préférence de 0,05 à 5% et plus préférentiellement encore de 0,1 à 3%.

Les compositions selon l'invention peuvent contenir en outre un ou plusieurs agents épaississants organiques.

Les agents épaississants organiques peuvent être choisis parmi les amides d'acides gras (diéthanol- ou monoéthanol-amide de coprah, monoéthanolamide d'acide alkyl éther carboxylique oxyéthyléné), les épaississants polymériques tels que les épaississants cellulosiques (hydroxyéthycellulose, hydroxypropylcellulose, carboxyméthylcellulose), la gomme de guar et ses dérivés (hydroxypropylguar), les gommes d'origine microbienne (gomme de xanthane, gomme de scléroglucane), les homopolymères réticulés d'acide acrylique ou d'acide acrylamidopropanesulfonique et les polymères associatifs (polymères amphiphiles comprenant des zones hydrophiles, et des zones hydrophobes à chaîne grasse capables, dans un milieu aqueux, de s'associer réversiblement entre eux ou avec d'autres molécules.).

Selon un mode de réalisation particulier, l'épaississant est polymérique et choisi parmi les épaississants cellulosiques (hydroxyéthycellulose, hydroxypropylcellulose, carboxyméthylcellulose), la gomme de guar et ses dérivés (hydroxypropylguar), les gommes d'origine microbienne (gomme de xanthane, gomme de scléroglucane), les homopolymères réticulés d'acide acrylique ou d'acide acrylamidopropanesulfonique.

En ce qui concerne les agents épaississants associatifs, on peut mettre en oeuvre un ou plusieurs polymères de nature non ionique ou ionique, de préférence anionique ou cationique.

Leur structure chimique comprend plus particulièrement au moins une zone hydrophile et au moins une zone hydrophobe. Par groupement hydrophobe, on entend un radical ou polymère à chaîne hydrocarbonée, saturée ou non, linéaire ou ramifiée, comprenant au moins 8 atomes de carbone, de préférence de 10 à 30 atomes de carbone, en particulier de 12 à 30 atomes de carbone et plus préférentiellement de 18 à 30 atomes de carbone.

Préférentiellement, le groupement hydrocarboné provient d'un composé monofonctionnel. A titre d'exemple, le groupement hydrophobe peut être issu d'un alcool gras tel que l'alcool stéarylique, l'alcool dodécylique, l'alcool décylique. Il peut également désigner un polymère hydrocarboné tel que par exemple le polybutadiène.

Parmi les polymères amphiphiles anioniques comportant au moins une chaîne grasse (hydrophobe), on peut citer :
- (I) les polymères comportant au moins un motif hydrophile, et au moins un motif éther d'allyle à chaîne grasse, plus particulièrement ceux dont le motif hydrophile est constitué par un monomère anionique insaturé éthylénique, avantageusement par un acide carboxylique vinylique et tout particulièrement par un acide acrylique ou un acide méthacrylique ou les mélanges de ceux ci, et dont le motif éther d'allyle à chaîne grasse correspond au monomère de formule (A) suivante :

   CH₂ = C R' CH₂ O Bₙ R(A)

   dans laquelle R' désigne H ou CH₃, B désigne le radical éthylèneoxy, n est nul ou désigne un entier allant de 1 à 100, R désigne un radical hydrocarboné choisi parmi les radicaux alkyle, arylalkyle, aryle, alkylaryle, cycloalkyle, comprenant de 8 à 30 atomes de carbone, de préférence 10 à 24, et plus particulièrement encore de 12 à 18 atomes de carbone. Un motif de formule (A) plus particulièrement préféré est un motif dans lequel R' désigne H, n est égal à 10, et R désigne un radical stéaryl (C₁₈).

Parmi ces polymères anioniques à chaîne grasse, on préfère les polymères formés à partir de 20 à 60% en poids d'acide acrylique et/ou d'acide méthacrylique, de 5 à 60% en poids de (méth)acrylates d'alkyles inférieurs, de 2 à 50% en poids d'éther d'allyl à chaîne grasse de formule (A), et de 0 à 1% en poids d'un agent réticulant qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.

Parmi ces derniers, on préfère tout particulièrement les terpolymères réticulés d'acide méthacrylique, d'acrylate d'éthyle, de polyéthylèneglycol (10 OE) éther d'alcool stéarylique (Steareth 10), notamment ceux vendus par la société ALLIED COLLOIDS sous les dénominations SALCARE SC 80 et SALCARE SC90 qui sont des émulsions aqueuses à 30% d'un terpolymère réticulé d'acide méthacrylique, d'acrylate d'éthyle et de steareth-10-allyl éther (40/50/10).
-(II) les polymères comportant au moins un motif hydrophile de type acide carboxylique insaturé oléfinique, et au moins un motif hydrophobe de type ester d'alkyl (C₁₀-C₃₀) d'acide carboxylique insaturé.

De préférence, ces polymères sont choisis parmi ceux dont le motif hydrophile de type acide carboxylique insaturé oléfinique correspond au monomère de formule (B) suivante : 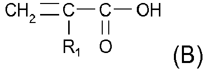 dans laquelle, R₁ désigne H ou CH₃ ou C₂H₅, c'est-à-dire des motifs acide acrylique, acide méthacrylique ou acide éthacrylique, et dont le motif hydrophobe de type ester d'alkyl (C₁₀-C₃₀) d'acide carboxylique insaturé correspond au monomère de formule (C) suivante : 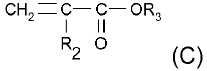 dans laquelle, R₂ désigne H ou CH₃ ou C₂H₅ (c'est-à-dire des motifs acrylates, méthacrylates ou éthacrylates) et de préférence H (motifs acrylates) ou CH₃ (motifs méthacrylates), R₃ désignant un radical alkyle en C₁₀-C₃₀, et de préférence en C₁₂-C₂₂.

Les esters d'alkyles (C₁₀-C₃₀) d'acides carboxyliques insaturés sont par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.

Parmi ce type de polymères anioniques à chaîne grasse, on utilisera plus particulièrement des polymères formés à partir d'un mélange de monomères comprenant :
(i) essentiellement de l'acide acrylique,
(ii) un ester de formule (C) décrite ci-dessus et dans laquelle R₂ désigne H ou CH₃, R₃ désignant un radical alkyle ayant de 12 à 22 atomes de carbone,
(iii) et un agent réticulant, qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.

Parmi ce type de polymères anioniques à chaîne grasse, on utilisera plus particulièrement ceux constitués de 95 à 60% en poids d'acide acrylique (motif hydrophile), 4 à 40% en poids d'acrylate d'alkyles en C₁₀-C₃₀ (motif hydrophobe), et 0 à 6% en poids de monomère polymérisable réticulant, ou bien ceux constitués de 98 à 96% en poids d'acide acrylique (motif hydrophile), 1 à 4% en poids d'acrylate d'alkyles en C₁₀-C₃₀ (motif hydrophobe), et 0,1 à 0,6% en poids de monomère polymérisable réticulant tel que ceux décrits précédemment.

Parmi lesdits polymères ci-dessus, on préfère tout particulièrement selon la présente invention, les produits vendus par la société GOODRICH sous les dénominations commerciales PEMULEN TR1, PEMULEN TR2, CARBOPOL 1382, et encore plus préférentiellement le PEMULEN TR1, et le produit vendu par la société S.E.P.P.I.C. sous la dénomination COATEX SX .
- (III) les terpolymères d'anhydride maléique / α-oléfine en C₃₀-C₃₈/ maléate d'alkyle tel que le produit (copolymère anhydride maléique / α-oléfine en C₃₀-C₃₈/ maléate d'isopropyle) vendu sous le nom PERFORMA V 1608 par la société NEWPHASE TECHNOLOGIES.
- (IV) les terpolymères acryliques comprenant :
   (a) 20% à 70% en poids d'un acide carboxylique à insaturation α,β-monoéthylénique,
   (b) 20 à 80% en poids d'un monomère à insaturation α,β-monoéthylénique non-tensio-actif différent de (a),
   (c) 0,5 à 60% en poids d'un mono-uréthane non-ionique qui est le produit de réaction d'un tensio-actif monohydrique avec un monoisocyanate à insaturation monoéthylénique,
   tels que ceux décrits dans la demande de brevet EP-A-0173109 et plus particulièrement un terpolymère acide méthacrylique /acrylate de méthyle/diméthyl métaisopropényl benzyl isocyanate d'alcool béhényle éthoxylé (40OE) en dispersion aqueuse à 25%.
- (V) les copolymères comportant parmi leurs monomères un acide carboxylique à insaturation α,β-monoéthylénique et un ester d'acide carboxylique à insaturation α,β-monoéthylénique et d'un alcool gras (C₈-C₃₀)oxyalkyléné.

Préférentiellement ces composés comprennent également comme monomère un ester d'acide carboxylique à insaturation α,β-monoéthylénique et d'alcool en C₁-C₄.

A titre d'exemple de ce type de composé on peut citer l'ACULYN 22 vendu par la société ROHM et HAAS, qui est un terpolymère acide méthacrylique/acrylate d'éthyle/méthacrylate de stéaryle oxyalkyléné.

Les polymères amphiphiles non ioniques à chaîne grasse (hydrophobe) sont choisis de préférence parmi :
-(1) les celluloses modifiées par des groupements comportant au moins une chaîne grasse, comme notamment ;
   - les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en C₈-C₂₂, comme le produit NATROSOL PLUS GRADE 330 CS (alkyles en C₁₆) vendu par la société AQUALON, ou le produit BERMOCOLL EHM 100 vendu par la société BEROL NOBEL,
   - celles modifiées par des groupes polyalkylène glycol éther d'alkyl phénol, tel que le produit AMERCELL POLYMER HM-1500 (polyéthylène glycol (15) éther de nonyl phénol) vendu par la société AMERCHOL.
-(2) les hydroxypropylguars modifiés par des groupements comportant au moins une chaîne grasse tel que le produit ESAFLOR HM 22 (chaîne alkyle en C₂₂) vendu par la société LAMBERTI, les produits RE210-18 (chaîne alkyle en C₁₄) et RE205-1 (chaîne alkyle en C₂₀) vendus par la société RHONE POULENC.
-(3) les copolymères de vinyl pyrrolidone et de monomères hydrophobes à chaîne grasse,
avec par exemple :
- les produits ANTARON V216 ou GANEX V216 (copolymère vinylpyrrolidone / hexadécène) vendu par la société I.S.P.
- les produits ANTARON V220 ou GANEX V220 (copolymère vinylpyrrolidone / eicosène) vendu par la société I.S.P.
   -(4) les copolymères de méthacrylates ou d'acrylates d'alkyles en C₁-C₆ et de monomères amphiphiles comportant au moins une chaîne grasse tels que par exemple le copolymère acrylate de méthyle/acrylate de stéaryle oxyéthyléné vendu par la société GOLDSCHMIDT sous la dénomination ANTIL 208.
   -(5) les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.
   -(6) les polymères à squelette aminoplaste éther possédant au moins une chaîne grasse, tels que les composés PURE THIX proposés par la société SUD-CHEMIE.
   -(7) les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

De préférence, les polyéthers polyuréthanes comportent au moins deux chaînes grasses hydrocarbonées, ayant de 8 à 30 atomes de carbone, séparées par une séquence hydrophile, les chaînes hydrocarbonées pouvant être des chaînes pendantes ou des chaînes en bout de séquence hydrophile.

A titre d'exemples de polyéthers polyuréthanes non ioniques à chaîne grasse utilisables dans l'invention, on peut utiliser le Rhéolate 205 à fonction urée vendu par la société RHEOX ou encore les Rhéolates 208 , 204 ou 212, ainsi que l'Acrysol RM 184, l'Aculyn ou Acrysol 44 et l'Aculyn ou Acrysol 46 de la société ROHM & HAAS [l'ACULYN 46 est un polycondensat de polyéthylèneglycol à 150 ou 180 moles d'oxyde d'éthylène, d'alcool stéarylique et de méthylène bis(4-cyclohexyl-isocyanate) (SMDI), à 15% en poids dans une matrice de maltodextrine (4%) et d'eau (81%); l'ACULYN 44 est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%)].

On peut également citer le produit ELFACOS T210 à chaîne alkyle en C₁₂-C₁₄ et le produit ELFACOS T212 à chaîne alkyle en C₁₈ de chez AKZO, ainsi que le produit DW 1206B de chez ROHM & HAAS à chaîne alkyle en C₂₀ et à liaison uréthanne, proposé à 20 % en matière sèche dans l'eau.

On peut aussi utiliser des solutions ou dispersions de ces polymères notamment dans l'eau ou en milieu hydroalcoolique. A titre d'exemple, de tels polymères on peut citer, le Rhéolate 255, le Rhéolate 278 et le Rhéolate 244 vendus par la société RHEOX. On peut aussi utiliser le produit DW 1206F et le DW 1206J proposés par la société ROHM & HAAS.

Les polyéthers polyuréthanes utilisables selon l'invention sont en particulier ceux décrits dans l'article de G. Fonnum, J. Bakke et Fk. Hansen - Colloid Polym. Sci 271, 380.389 (1993).

Les polymères amphiphiles cationiques comportant au moins une chaîne grasse (hydrophobe) utilisés peuvent notamment être choisis parmi les dérivés de cellulose quaternisée, les polyuréthanes cationiques, les polyvinyllactames cationiques, et de préférence, parmi les dérivés de cellulose quaternisée.

A tire d'exemple de polymères de ce type, on peut citer en particulier :
- les celluloses quaternisées modifiées par des groupements comportant au moins une chaîne grasse, tels que les groupes alkyle, arylalkyle, alkylaryle comportant au moins 8 atomes de carbone, ou des mélanges de ceux-ci,
- les hydroxyéthylcelluloses quaternisées modifiées par des groupements comportant au moins une chaîne grasse, tels que les groupes alkyle, arylalkyle, alkylaryle comportant au moins 8 atomes de carbone, ou des mélanges de ceux-ci.

Les radicaux alkyle portés par les celluloses ou les hydroxyéthylcelluloses quaternisées ci-dessus comportent de préférence de 8 à 30 atomes de carbone. Les radicaux aryle désignent de préférence les groupements phényle, benzyle, naphtyle ou anthryle.

On peut indiquer comme exemples d'alkylhydroxyéthylcelluloses quaternisées à chaînes grasses en C₈-C₃₀, les produits QUATRISOFT LM 200, QUATRISOFT LM-X 529-18-A, QUATRISOFT LM-X 529-18B (alkyle en C₁₂) et QUATRISOFT LM-X 529-8 (alkyle en C₁₈) commercialisés par la société AMERCHOL et les produits CRODACEL QM, CRODACEL QL (alkyle en C₁₂) et CRODACEL QS (alkyle en C18) commercialisés par la société CRODA.

La teneur en polymères épaississants, s'ils sont présents, varie habituellement de 0,05 % à 5 % en poids, par rapport au poids de la composition de coloration.

Selon un mode de réalisation particulièrement avantageux, la composition tinctoriale comprend un ou plusieurs agents tensioactifs. Ces derniers peuvent être indifféremment choisis, seuls ou en mélanges, parmi des tensioactifs anioniques, amphotères, non ioniques, zwittérioniques et cationiques.

En ce qui concerne les tensioactifs anioniques, on utilise habituellement les sels, en particulier les sels de métaux alcalins tels que les sels de sodium, les sels d'ammonium, les sels d'amines, les sels d'aminoalcools ou les sels de métaux alcalino-terreux, par exemple, de magnésium, des composés suivants, seuls ou en mélange :
- les alkylsulfates, les alkyléthersulfates, les alkylamidoéthersulfates, les alkylarylpolyéthersulfates ;
- les alkylsulfonates, les alkylamidesulfonates, les alkyl-arylsulfonates ;
- les alkylsulfosuccinates, les alkyléthersulfosuccinates, les alkylamide-sulfosuccinates ;
- les alkylsulfoacétates ;
- les acylsarconisates ; et les acylglutamates :
- les esters d'alkyle et d'acides polyglycoside-carboxyliques tels que les glucoside-citrates d'alkyle, les polyglycoside-tartrates d'alkyle, et les polyglycoside-sulfosuccinates d'alkyle ;
- les alkylsulfosuccinamates ;
- les acyliséthionates, les N-acyltaurates ; les acyllactylates ;
- les acides d'alkyl-D-galactoside uroniques ;
- les acides alkyléther-carboxyliques polyoxyalkylénés, les acides alkylaryléther-carboxyliques polyoxyalkylénés, les acides alkylamidoéther carboxyliques polyoxyalkylénés;
le groupe alkyle ou acyle (RCO-) de ces composés comportant de 10 à 24 atomes de carbone et le groupe aryle désignant de préférence un groupe phényle ou benzyle ; le nombre de groupements oxyalkylénés, et de préférence oxyéthylénés, est compris entre 2 et 50.

Pour ce qui concerne les tensioactifs non ioniques, ces derniers peuvent être avantageusement choisis parmi les composés suivants, seuls ou en mélange :
- les alcools gras polyéthoxylés, polypropoxylés ou polyglycérolés,
- les alpha-diols polyéthoxylés, polypropoxylés ou polyglycérolés,
le nombre de groupements oxyde d'éthylène ou oxyde de propylène allant de 2 à 50 ; le nombre de groupements glycérol allant de 2 à 30 ;
- les condensats d'oxyde d'éthylène et d'oxyde de propylène sur des alcools gras ;
- les amides gras polyéthoxylés ayant de 2 à 30 moles d'oxyde d'éthylène ;
- les amides gras polyglycérolés comportant de 1 à 5 groupements glycérol ;
- les esters éthoxylés d'acides gras du sorbitane ayant de 2 à 30 moles d'oxyde d'éthylène, les esters d'acides gras du saccharose ;
- les alkylpolyglucosides, les dérivés de N-alkylglucamine ;
ces composés comprenant au moins une chaîne alkyle ou alcényle comprenant 10 à 24 atomes de carbone.
- les copolymères d'oxyde d'éthylène et d'oxyde de propylène.

Les tensioactifs cationiques entrant dans la composition selon l'invention, peuvent notamment être choisis parmi les composés suivants, seuls ou en mélange :
- les amines grasses primaires, secondaires ou tertiaires éventuellement polyéthoxylées (2 à 30 moles d'oxyde d'éthylène) et leurs sels
- les sels d'ammonium quaternaire comprenant ou non une ou plusieurs fonctions ester tels que les chlorures ou les bromures de tétraalkylammonium, d'alkylamidoalkyltrialkylammonium, de trialkylbenzyl-ammonium, de trialkylhydroxyalkyl ammonium, d'alkylpyridinium, et les diesters quaternaires.
- les dérivés d'alkyl-imidazoline ;
ces composés comprenant au moins une chaîne alkyle comprenant 10 à 24 atomes de carbone.

Enfin, les tensioactifs amphotères peuvent être choisis parmi les composés suivants, seuls ou en mélange :
- les dérivés d'amines aliphatiques secondaires ou tertiaires, dans lesquels le groupe aliphatique est une chaîne linéaire ou ramifiée comportant 10 à 24 atomes de carbone et comprenant au moins un groupe anionique hydrosolubilisant tel que, par exemple, un groupe carboxylate, sulfonate, sulfate, phosphate ou phosphonate,
- les alkylbétaïnes, les sulfobétaïnes, les alkylamidoalkyl(C₆-C₈)bétaïnes, les alkylamidoalkyl(C₆-C₈)sulfobétaïnes ;
ces composés comprenant une au moins chaîne alkyle comprenant de 10 à 24 atomes de carbone.

De préférence, les tensioactifs sont non ioniques, anioniques ou amphotères et de manière encore plus préférée, non ioniques.

Habituellement, les agents tensioactifs représentent une quantité comprise entre 0,01 et 50% en poids, de préférence entre 0,1 et 25% en poids par rapport au poids de la composition.

La composition tinctoriale conforme à l'invention peut également renfermer divers adjuvants utilisés classiquement dans les compositions pour la teinture des cheveux, tels que par exemple des polymères anioniques, non ioniques, amphotères, zwitterioniques différents des épaississants mentionnés auparavant, ou leurs mélanges ; des agents épaississants minéraux comme notamment les argiles ; des agents antioxydants tels que par exemple l'acide ascorbique, l'acide érythorbique ; des agents réducteurs avec entre auters le sulfite, bisulfite ou métabisulfite d'ammonium, le thiolactate d'ammonium ; des agents de pénétration, des agents séquestrants comme l'éthylènediamine tétraacétique ou ses sels ; des parfums ; des agents matifiants avec par exemple les oxydes de titane ; des tampons ; des agents dispersants ; des agents filmogènes ; des céramides ; des acides carboxyliques notamment aromatiques comme l'acide benzoïque ; et des agents conservateurs.

Les adjuvants ci dessus sont en général présents en quantité comprise pour chacun d'eux entre 0,01 et 20% en poids par rapport au poids de la composition.

Le milieu cosmétiquement acceptable de la composition, qui est un milieu approprié pour la coloration des fibres kératiniques humaines, comprend de préférence de l'eau et éventuellement un ou plusieurs solvants additionnels différents du 3-phényl-1-propanol.

A titre d'exemples de tels solvants additionnels, on peut par exemple citer, les alcanols, linéaires ou ramifiés, en C₂-C₄, tels que l'éthanol et l'isopropanol ; les polyols et éthers de polyols comme le 2-butoxyéthanol, l'hexylène glycol, le propylèneglycol, le dipropylèneglycol, le monométhyléther de propylèneglycol, le monoéthyléther et le monométhyléther du diéthylèneglycol, le glycérol ainsi que les alcools aromatiques comme l'alcool benzylique, le benzyloxyéthanol ou le phénoxyéthanol, et leurs mélanges.

Le ou les solvants additionnels peuvent être présents dans des proportions allant de préférence de 0,1 à 40% en poids par rapport au poids total de la composition tinctoriale, et encore plus préférentiellement de 5 à 30% en poids.

Il est à noter que la quantité d'eau dans la composition selon l'invention est de préférence supérieure à 10 % en poids par rapport au poids de la composition, et plus avantageusement supérieure ou égale à 25% en poids. De préférence, la teneur en eau est comprise entre 25 et 98% en poids par rapport au poids de la composition.

Le pH de la composition selon l'invention peut être compris entre 2 et 13. Il est de préférence compris entre 6 et 11.

Au cas où la composition ne comprend pas de colorant d'oxydation, alors le pH de la composition peut être compris entre 2 et 13. Il est plus précisément compris entre 2 et 11, de préférence, il est inférieur à 7, et mieux entre 2 et 6.

Il peut être ajusté à la valeur désirée au moyen d'un ou plusieurs agents acidifiants ou d'un ou plusieurs agents alcalinisants habituellement utilisés dans le domaine.

Parmi les agents acidifiants, on peut citer à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, les carbonates alcalins, les alcanolamines telles que les mono-, di- et triéthanolamines ainsi que leurs dérivés, les hydroxydes de sodium ou de potassium et les composés de formule suivante : 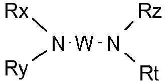 dans laquelle W est un reste propylène éventuellement substitué par un groupement hydroxyle ou un radical alkyle en C₁-C₆ ; Rx, Ry, Rz et Rt, identiques ou différents, représentent un atome d'hydrogène, un radical alkyle en C₁-C₆ ou hydroxyalkyle en C₁-C₆.

La composition selon l'invention peut comprendre également un ou plusieurs agents oxydants. On parle dans ce cas de composition prête à l'emploi.

En particulier, la composition prête à l'emploi est obtenue par mélange extemporané avant l'application, d'une composition précédemment décrite, avec au moins une composition comprenant un ou plusieurs agents oxydants.

L'agent oxydant est choisi de préférence parmi le peroxyde d'hydrogène, le peroxyde d'urée, les bromates ou ferricyanures de métaux alcalins, les sels peroxygénés comme par exemple les persulfates, les perborates et les percarbonates de métaux alcalins ou alcalino-terreux, comme le sodium, le potassium, le magnésium.

L'utilisation du peroxyde d'hydrogène est particulièrement préférée.

Cet agent oxydant est avantageusement constitué du peroxyde d'hydrogène en solution aqueuse (eau oxygénée) dont le titre peut varier, plus particulièrement, de 1 à 40 volumes, et encore plus préférentiellement de 5 à 40 volumes.

Les compostions selon l'invention peuvent résulter du mélange extemporané de plusieurs compositions.

De préférence les fibres kératiniques humaines sont les cheveux.

Des exemples concrets mais non limitatifs de l'invention vont maintenant être présentés

### Exemples 1

On prépare les compositions A, B et C suivantes (les proportions sont indiquées en % en poids) :

| | **A Invention** | **B Comparative** | **C Comparative** |
|---|---|---|---|
| 3-phényl-1-propanol | 0,5% MA | - | - |
| 2-phényl-1-éthanol | - | 0,5 % MA | - |
| Alcool benzylique | - | - | 0,5% MA |
| Ethanol | 15% | | |
| Disperse Red 13 | 0.5% | | |
| Agent de pH | qsp pH=2,7 | | |
| Parfum | qs | | |
| Eau | qsp 100 | | |

Des mèches de cheveux à 90% de cheveux blancs naturels sont traitées par les compositions A, B ou C.

Après un temps de pause de 20 minutes à température ambiante, les mèches sont essorées, rincées, shampouinées et séchées.

On constate qu'avec le colorant Disperse Red 13, la composition de l'invention contenant le 3-phényl-1-propanol (Composition A) permet de colorer les cheveux naturels dans une couleur intense.

On constate également que la coloration est plus intense qu'avec d'autres solvants alcooliques aromatiques tels que l'alcool benzylique ou le 2-phényl-1-éthanol.

### Exemples 2

On prépare les compositions A, B et C suivantes (les proportions sont indiquées en % en poids) :

| | **A Invention** | **B Comparative** | **C Comparative** |
|---|---|---|---|
| 3-phenyl-1-propanol | 0,5% MA | - | - |
| 2-phenyl-1-ethanol | - | 0,5 % MA | - |
| Alcool benzylique | - | - | 0,5% MA |
| Ethanol | 15% | | |
| Agent de pH | qsp pH=2,7 | | |
| Parfum | qs | | |
| Eau | qsp 100 | | |

On mélange chacune des compositions A, B et C avec de l'orcéine [NATURAL RED 28 / Cl 758600] de telle sorte que chaque mélange résultant comprenne 0,5 % en poids de colorant.

On applique ensuite chacun des mélanges sur des mèches de cheveux à 90% de cheveux blancs naturels.

Le temps d'application est de 20 minutes à température ambiante. A l'issue de ce temps de pause, les mèches sont essorées, rincées, shampouinées puis séchées.

On constate que la composition conforme à l'invention permet d'obtenir une coloration plus intense que dans le cas des compositions comparatives.

## Revendications

1. Composition tinctoriale comprenant dans un milieu cosmétiquement acceptable, un ou plusieurs colorants choisis parmi les colorants directs synthétiques cationiques ou non ioniques, les colorants naturels ou leurs mélanges; et le 3-phényl-1-propanol, et comprenant de l'eau et un ou plusieurs solvants organiques additionnels différents du 3-phényl-1-propanol.

2. Composition selon la revendication précédente, **caractérisée en ce que** la teneur en 3-phényl-1-propanol représente de 0,05 à 50% et de préférence de 0,1 à 30% en poids, par rapport au poids de la composition.

3. Composition selon la revendication précédente, **caractérisée en ce que** le ou les colorants directs sont choisis parmi les colorants directs azoïques ; méthiniques ; carbonyles ; aziniques ; nitrés (hétéro)aryle ; tri-(hétéro)aryle méthanes ; seuls ou en mélanges.

4. Composition selon l'une quelconque des revendications précédentes, **caractérisée en ce que** le ou les colorants directs sont choisis parmi les colorants nitrés de la série benzénique ; les colorants directs azoïques ; méthiniques ; azométhiniques avec plus particulièrement les diazacarbocyanines et leurs isomères et les tétraazacarbocyanines (tétraazapentaméthines) ; les colorants directs quinoniques et en particulier anthraquinoniques, naphtoquinoniques ou benzoquinoniques ; les colorants directs aziniques ; xanthéniques ; triarylméthaniques ; indoaminiques ; indigoïdes ; phtalocyanines et porphyrines ; seuls ou en mélanges.

5. Composition selon l'une quelconque des revendications précédentes, **caractérisée en ce que** le ou les colorants directs sont choisis parmi les colorants directs cationiques monochromophoriques de types azoïques ; méthines ; azométhines avec les diazacarbocyanines et leurs isomères, les tétraazacarbocyanines (tétraazapentaméthines) ; anthraquinoniques ; seuls ou en mélange ; et parmi les colorants non ioniques on préfère tout particulièrement les composés présentant un LogP supérieur ou égal à 2.

6. Composition selon l'une quelconque des revendications précédentes, **caractérisée en ce que** le ou les colorants directs sont choisis parmi les composés de formules (A1) à (A6) et les colorants non ioniques de log P supérieur ou égal à 2 :

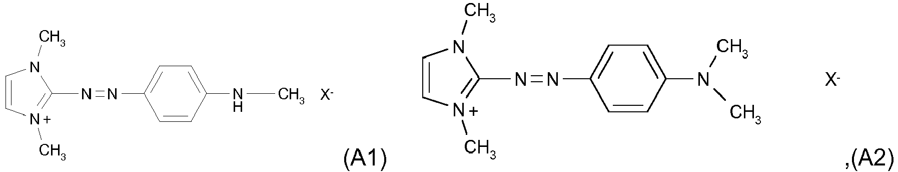

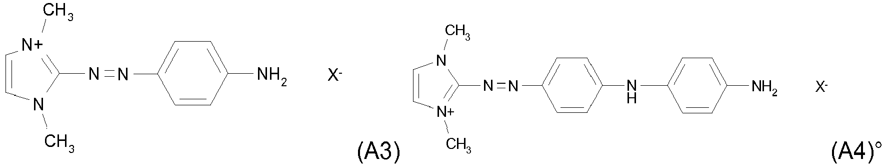

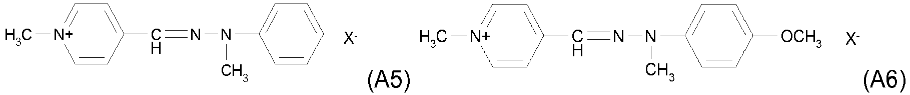

7. Composition selon la revendication précédente, **caractérisée en ce que** le ou les colorants naturels sont choisis parmi la lawsone, la juglone, l'alizarine, la purpurine, l'acide carminique, l'acide kermésique, la purpurogalline, l'anthragallol la protocatéchaldéhyde, l'indigo, l'isatine, la curcumine, la spinusoline, les chlorophylles, les chloropyllines, les orcéines, l'hématéine, l'hématoxyline, la braziline, la braziléine, les colorants du carthame, les flavonoïdes, les anthocyanes, les caroténoïdes, les tanins, le sorgho et le carmin de cochenille, ainsi que les extraits ou décoctions contenant ces colorants naturels et notamment les extraits à base de henné, ou leurs mélanges.

8. Composition selon l'une quelconque des revendications précédentes, **caractérisée en ce qu'**elle comprend un ou plusieurs agents épaississants organiques, de préférence polymériques, et/ou un ou plusieurs agents tensioactifs.

9. Composition selon l'une quelconque des revendications précédentes, **caractérisée en ce qu'**elle comprend un ou plusieurs agents oxydants.

## Patentansprüche

1. Färbezusammensetzung, umfassend, in einem kosmetisch annehmbaren Medium, einen oder mehrere Farbstoffe, ausgewählt aus kationischen oder nicht ionischen synthetischen direkten Farbstoffen, natürlichen Farbstoffen oder ihren Mischungen; und 3-Phenyl-1-propanul, und umfassend Wasser oder ein oder mehrere zusätzliche organische Lösungsmittel, die von 3-Phenyl-1-propanol verschieden sind.

2. Zusammensetzung nach dem vorhergehenden Anspruch, **dadurch gekennzeichnet, dass** der Gehalt an 3-Phenyl-1-propanol von 0,05 bis 50 Gew.-% und vorzugsweise von 0,1 bis 30 Gew.-%, bezogen auf das Gewicht der Zusammensetzung, beträgt.

3. Zusammensetzung nach dem vorhergehenden Anspruch, **dadurch gekennzeichnet, dass** der oder die direkten Farbstoffe ausgewählt sind aus direkten Azo-, Methin-, Carbonyl-, Azin-, Nitro(hetero)aryl-, Tri(hetero)arylmethan-Farbstoffen, allein oder in Mischungen.

4. Zusammensetzung nach einem der vorhergehenden Ansprüche,
**dadurch gekennzeichnet, dass** der oder die direkten Farbstoffe ausgewählt sind aus Nitro-Farbstoffen der Benzol-Serie, direkten Azo-, Methin-, Azomethin-Farbstoffen mit insbesondere Diazacarbocyaninen und ihren Isomeren und Tetraazacarbocyaninen (Tetraazapentamethinen), direkten Chinon- und insbesondere Anthrachinon-, Naphtochinon- oder Benzochinon-Farbstoffen, direkten Azin-, Xanthen-, Triarylmethan-, Indoamin-, Indigoid-, Phthalocyanin- und Porphyrin-Farbstoffen, allein oder in Mischungen.

5. Zusammensetzung nach einem der vorhergehenden Ansprüche,
**dadurch gekennzeichnet, dass** der oder die direkten Farbstoffe ausgewählt sind aus monochromophoren kationischen direkten Farbstoffen vom Azo-Typ, Methin-, Azomethin-Farbstoffen mit Diazacarbocyaninen und ihren Isomeren, Tetraazacarbocyaninen (Tetraazapentamethinen), Anthrachinon-Farbstoffen, allein oder in Mischung, und unter der nichtionischen Farbstoffen insbesondere jene Verbindungen bevorzugt werden, die einen LogP größer oder gleich 2 aufweisen.

6. Zusammensetzung nach einem der vorhergehenden Ansprüche,
**dadurch gekennzeichnet, dass** der oder die direkten Farbstoffe ausgewählt sind aus Verbindungen mit den Formeln (A1) bis (A6) und nichtionischen Farbstoffen mit einem LogP größer oder gleich 2:

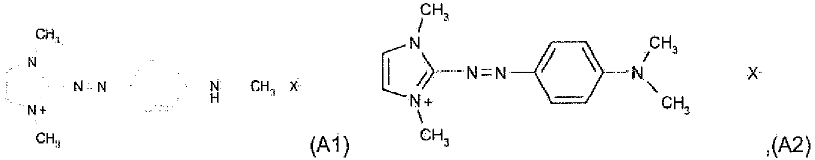

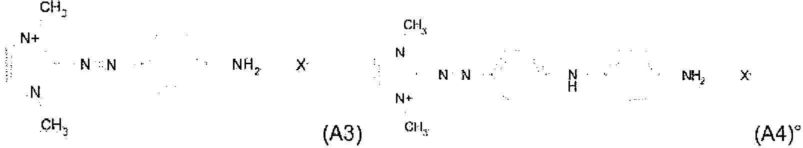

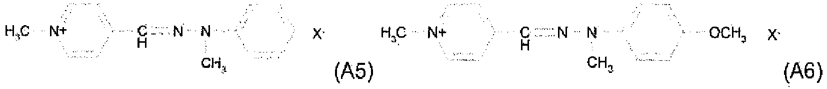

7. Zusammensetzung nach dem vorhergehenden Anspruch, **dadurch gekennzeichnet, dass** der oder die natürlichen Farbstoffe ausgewählt sind aus Lawson, Juglon, Alizarin, Purpurin, Karminsäure, Kermesinsäure, Purpurogallin, Anthragallol, Protocatechaldehyd, Indigo, Isatin, Curcumin, Spinusolin, Chlorophyllen, Chlorphyllinen, Orceinen, Hematein, Hematoxylin, Brazilin, Brazilein, Farbstoffen von Cartham, Flavonoiden, Anthocyanen, Carotenoiden, Taninen, Sorgho und Cochenillecarmin, sowie Extrakten oder Dekokten, enthaltend natürliche Farbstoffe, und insbesondere Extrakten auf Henna-Basis oder ihren Mischungen.

8. Zusammensetzung nach einem der vorhergehenden Ansprüche,
**dadurch gekennzeichnet, dass** sie ein oder mehrere organische, vorzugsweise polymere, Verdickungsmittel und/oder einen oder mehrere grenzflächenaktive Stoffe umfasst.

9. Zusammensetzung nach einem der vorhergehenden Ansprüche,
**dadurch gekennzeichnet, dass** sie ein oder mehrere Oxidationsmittel umfasst.

## Claims

1. Dye composition comprising, in a cosmetically acceptable medium, one or more dyes chosen from cationic or nonionic synthetic direct dyes and natural dyes, or mixtures thereof; and 3-phenyl-1-propanol, and comprising water and one or more additional organic solvents other than 3-phenyl-1-propanol.

2. Composition according to the preceding claim, **characterized in that** the content of 3-phenyl-1-propanol represents from 0.05% to 50% by weight and preferably from 0.1% to 30% by weight relative to the weight of the composition.

3. Composition according to the preceding claim, **characterized in that** the direct dye(s) are chosen from the following direct dyes: azo dyes; methine dyes; carbonyl dyes; azine dyes; nitro(hetero)aryl dyes; tri(hetero)arylmethane dyes; alone or as mixtures.

4. Composition according to any one of the preceding claims, **characterized in that** the direct dye(s) are chosen from nitrobenzene dyes; azo direct dyes; methine direct dyes; azomethine direct dyes, with, more particularly, diazacarbocyanins and isomers thereof and tetraazacarbocyanins (tetraazapentamethines) ; quinone direct dyes, and in particular anthraquinone, naphthoquinone or benzoquinone dyes; azine direct dyes; xanthene direct dyes; triarylmethane direct dyes; indoamine direct dyes; indigoid direct dyes; phthalocyanine and porphyrin direct dyes; alone or in mixtures.

5. Composition according to any one of the preceding claims, **characterized in that** the direct dye(s) are chosen from monochromophoric cationic direct dyes of the following types: azo dyes; methine dyes; azomethine dyes with diazacarbocyanins and isomers thereof, and tetraazacarbocyanins (tetraazapentamethines) ; anthraquinone dyes; alone or as a mixture; and among the nonionic dyes, compounds with a logP of greater than or equal to 2 are most particularly preferred.

6. Composition according to any one of the preceding claims, **characterized in that** the direct dye(s) are chosen from the compounds of formulae (A1) to (A6) and nonionic dyes with a logP of greater than or equal to 2:

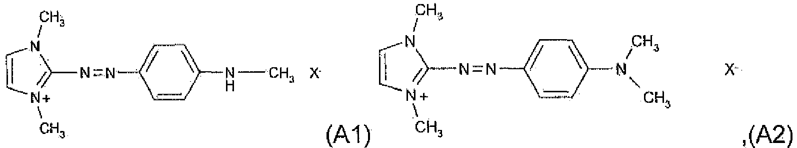

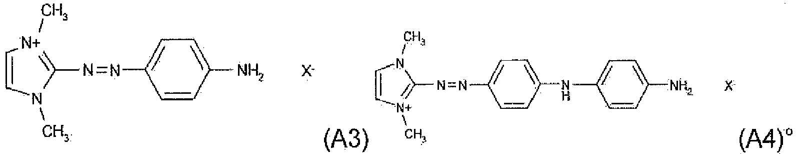

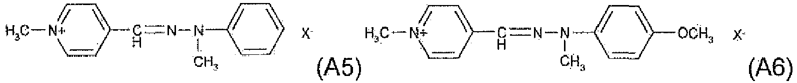

7. Composition according to the preceding claim, **characterized in that** the natural dye(s) are chosen from lawsone, juglone, alizarin, purpurin, carminic acid, kermesic acid, purpurogallin, anthragallol, protocatechaldehyde, indigo, isatin, curcumin, spinulosin, chlorophylls, chlorophyllines, orceins, hematin, hematoxylin, brazilin, brazileine, safflower colorants, flavonoids, anthocyans, carotenoids, tannins, sorghum and cochineal carmine, and also extracts or decoctions containing these natural dyes and especially henna-based extracts, or mixtures thereof.

8. Composition according to any one of the preceding claims, **characterized in that** it comprises one or more organic thickeners, preferably polymeric thickeners, and/or one or more surfactants.

9. Composition according to any one of the preceding claims, **characterized in that** it comprises one or more oxidizing agents.
